# EUROPEAN PATENT APPLICATION

(11) **EP 2 727 538 A2**
(43) Date of publication of application: **07.05.2014**
(21) Application number: 13187934.8
(22) Date of filing: 09.10.2013
(51) Int. Cl.: A61B 17/00

(54) **Device and method for dispensing a liquid and a gas**

(30) Priority: 31.10.2012 US 201261720694 P; 13.03.2013 US 201313798448
(71) Applicant: Nordson Corporation, Westlake, Ohio 44145-1119 (US)
(72) Inventor: Anderson, Benjamin, Eau Claire, WI 54701 (US); Lou, Huadong, Plymouth, MN 55446 (US); Hoogenakker, Jon E., Inver Grove Heights, MN 55077 (US); Kirk, Thomas A., Hastings, Minnesota 55033 (US)
(74) Representative: Eisenführ Speiser

(57) **Abstract**

A device (10) and method for dispensing a liquid and a gas from a tip includes a support structure (18), a control unit (26), and a drive unit (32). The liquid is held within a container (30a,30b). The support structure is adapted to hold the container. The control unit directs the drive unit to expel liquid from the container according to a predetermined rate. The control unit further directs the drive unit according to first, second, and third modes of operation. During the first mode, neither gas nor liquid is dispensed from the device. During the second mode, only gas is dispensed from the device. During the third mode, both gas and liquid are dispensed from the device. Furthermore, the control unit is only selectable and operable by a user through the first, second, and third modes in a sequential manner.

## Description

### Cross-Reference to Related Application

This application claims the priority of Application Serial No. 61/720,694 filed October 31, 2012 (pending), the disclosure of which is hereby incorporated by reference herein.

### Technical Field

The present invention relates generally to a device and method for dispensing a liquid and a gas, and more particularly, to a device and method for dispensing reactive liquids and gas for use in a medical procedure.

### Background

Generally, it is known to dispense reactive liquids in the form of sprayed droplets for use in various fields. For example, a plurality of reactive liquids may be sprayed under the influence of pressurized gas to disperse and dispense the droplets on the human body or within the human body during a medical procedure. For instance, two reactive fluids may be sprayed onto an anatomical site for reducing the flow of blood by hemostatic clotting or creating tissue barriers to prevent anatomical tissues from adhering together during and/or after the medical procedure. These reactive materials are typically biomaterials, such as fibrin and thrombin. Ideally, these reactive liquids are isolated prior to being discharged. The reactive liquids mix and react once discharged at the anatomical site.

Reactive liquids used in medical procedures and gas are frequently dispensed from a tip connected to an applicator. The applicator traditionally employs two independently operable control units for respectively dispensing the reactive liquids and gas. Accordingly, a first control unit selectively dispenses the gas, and a second control unit selectively dispenses the reactive liquids. The first control unit is typically a switch operatively connected to a valve for dispensing the gas. The switch is manipulated by the foot of a user. In contrast, the second control unit is a trigger manipulated by the user's hand for dispensing the liquid. The flow rate of the liquid dispensed from the tip is in direct response to the force with which the user manipulates the trigger. Thus, different users tend to dispense liquid at differing rates, which may create inconsistencies among various users. Furthermore, due to the inherent complexity of operating the applicator simultaneously by foot and hand, the user must be well-trained and skilled to properly dispense gas and reactive liquids during medical procedures.

Best practices for applying the gas and the reactive liquids to anatomical sites further complicates the foot and hand operation of the applicator. First, the user preferably manipulates the first control unit to discharge gas for generally drying the anatomical site. This prepares the anatomical site for application of the reactive liquids. The user then continues to manipulate the first control unit while also manipulating the second control unit for dispensing the reactive liquids as sprayed droplets. Failure to properly manipulate the first and second control units will result in either too much or too little application of the reactive liquids.

Once the reactive liquids have been applied to the anatomical site, portions of the reactive liquid may remain present in and around the tip. These remaining reactive liquids tend to clog the tip and diminish its useful life. Thus, after dispensing the reactive liquids, the user manipulates the first control unit yet again to dispense only gas from the tip. The gas then blows the reactive liquids free from the tip to prevent clogging.

There is a need for a device and method for use in dispensing at least one liquid and a gas, such as during a medical procedure, to address present challenges such as those discussed above.

### Summary

One exemplary embodiment of a device dispenses a liquid and a gas from a tip. The liquid is held within a container. The device generally includes a support structure adapted for holding the container, a drive unit, and a control unit. The support structure has a liquid passage, a gas passage, and a gas supply conduit. The gas supply conduit is in selective fluid communication with the gas passage for selectively discharging the gas from the gas passage and into a cannula having the tip. The liquid passage is adapted for fluid communication with the container for selectively discharging the liquid from the liquid passage and into the cannula. The liquid and the gas are adapted to dispense from the tip to form droplets of the liquid for appropriate application.

The control unit and the drive unit are connected to the support structure. The drive unit is configured to actuate for selectively expelling the liquid from the container and discharging the liquid from the liquid passage. The control unit is operatively connected to the drive unit and the gas supply conduit to allow selection and operation according to a first, second, and third modes of operation. The first mode neither actuates the drive unit nor fluidly connects the gas supply conduit to the gas passage. The second mode fluidly connects the gas supply conduit only to the gas passage. The third mode actuates the drive unit and fluidly connects the gas supply conduit to the gas passage. More particularly, the control unit is operable by a user such that the first, second, and third modes can only be selected and operated in a sequential manner. Furthermore, the control unit directs the drive unit to expel liquid according to a predetermined rate.

The device also includes a cannula in fluid communication with the liquid passage. The cannula has a proximal end connected to the liquid passage. The cannula has a distal end having a tip attached thereto that is configured for dispensing the liquid and the gas. As such, the liquid is dispensed from the tip according to the predetermined rate.

In one aspect, the device further includes a valve and the drive unit is a pneumatic drive unit. The pneumatic drive unit is configured to actuate via the gas for selectively expelling the liquid from the container and discharging the liquid from the liquid passage. The valve is connected to the support structure and in fluid communication with the gas supply conduit, the gas passage, and the pneumatic drive unit. The valve is movable between a first position, a second position, and a third position. The first position disconnects the pneumatic drive unit from the gas supply conduit. The second position fluidly connects only the gas passage to the gas supply conduit. The third position fluidly connects both the pneumatic drive unit and the gas passage to the gas supply conduit. More particularly, the valve is operable by a user such that the first, second, and third modes can only be selected and operated in a sequential manner.

The valve further includes a valve chamber and a spool valve. The valve chamber is in fluid communication with the gas supply conduit, the gas passage, and the pneumatic drive unit. In addition, the valve chamber has a longitudinal axis. The spool valve is positioned within the chamber and slidable between first, second, and third positions along the longitudinal axis. A vent passage also extends from the valve to an exterior environment. The first and second positions of the valve fluidly connect the vent passage to the pneumatic drive unit for relieving excess gas from the pneumatic drive unit to the exterior environment. The third position of the valve fluidly disconnects the pneumatic drive unit from the valve passage.

In another aspect, the pneumatic drive unit further includes a cylinder, a piston positioned within the cylinder, and an actuator operatively connected to the piston for actuating the actuator. The cylinder is in fluid communication with the gas supply conduit, and the piston is adapted to slide along the cylinder under the influence of pressurized gas from the gas supply conduit. Thus, gas from the gas supply conduit is adapted to actuate the actuator, which, in turn, moves the container. In addition, the vent passage is in fluid communication with the cylinder and the exterior environment. The vent passage is adapted to selectively vent excess gas from the cylinder to the exterior environment so that the container actuates according to the predetermined rate.

The support structure further comprises a plunger having the liquid passage extending therethrough. As the container is actuated by the actuator, the plunger is adapted for being inserted into the container while the container moves. More particularly, the plunger engages the plug as the container moves, which forces the plug into the container to expel the liquid from the liquid passage.

In use, the liquid and the gas are dispensed from the tip for use in the medical procedure by selectively manipulating the control unit to simultaneously dispense the liquid and the gas from the tip. The liquid is dispensed according to the predetermined rate regardless of the rate at which the control unit is selectively manipulated. Specifically, the control unit may be manipulated according to the first, second, and third modes of operation. Neither liquid nor gas is dispensed from the tip during the first mode. Only gas is dispensed from the tip during the second mode. Both gas and liquid are simultaneously dispensed from the tip during the third mode. Only the single control unit is used to dispense the liquid and the gas according to each of the first, second, and third modes.

Various additional objectives, advantages, and features of the invention will be appreciated from a review of the following detailed description of the illustrative embodiments taken in conjunction with the accompanying drawings.

### Brief Description of the Drawings

The accompanying drawings, which are incorporated in and constitute a part of this specification, illustrate embodiments of the invention and, together with a general description of the invention given above, and the detailed description given below serve to explain the invention.

FIG. 1 is a perspective view of a device according to an embodiment of the invention.

FIG. 2A is a cross-sectional view of FIG. 1 taken along section line 2-2 according to a first mode of operation.

FIG. 2B is a cross-sectional view of FIG. 1 taken along section line 2-2 according to a second mode of operation.

FIG. 2C is a cross-sectional view of FIG. 1 taken along section line 2-2 according to a third mode of operation.

FIG. 3 is a perspective view of another applicator according to an embodiment of the invention.

### Detailed Description

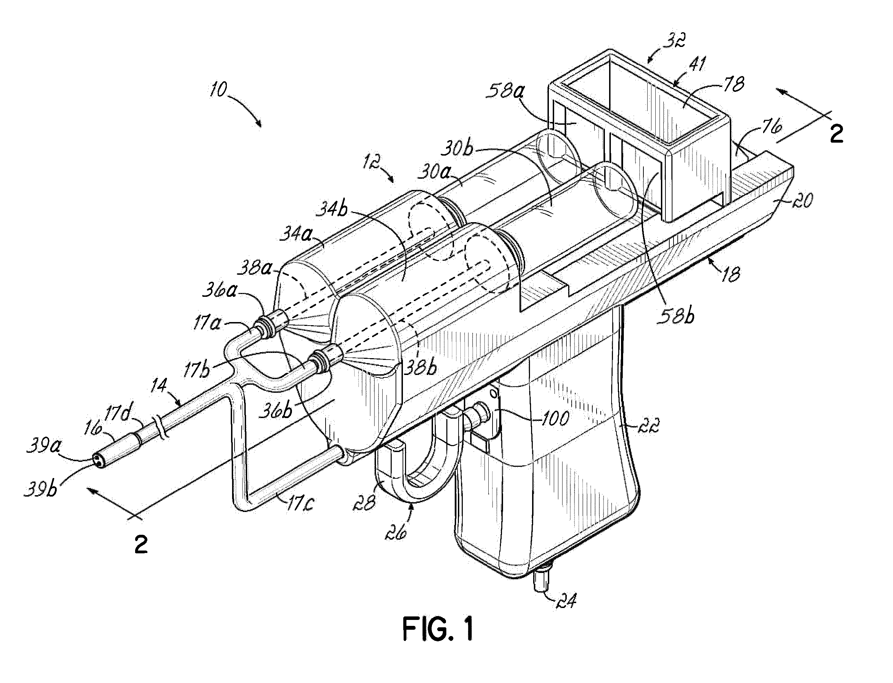

With reference to FIG. 1, an embodiment of the device 10 for dispensing a reactive liquid and a gas includes an applicator 12, particularly for use during medical procedures. A cannula 14 is connected to both the applicator 12 and a tip 16 configured for dispensing the liquid and gas. More particularly, the cannula 14 includes a pair of proximal ends 17a, 17b, a gas supply end 17c, and a distal end 17d. The distal end 17d is connected to the tip 16 while the proximal ends 17a, 17b and the gas supply end 17c are connected to the applicator 12. The cannula 14 may be rigid, flexible, or flexible and steerable. The cannula 14 is adapted to deliver two liquids and the gas separately to the tip 16 for spraying droplets during applications of any desired type, e.g., medical procedures. The liquids mix while exiting the tip 16 and form into droplets under the influence of the adjacently dispensed gas. Such medical procedures for use with the device 10 may include topical applications, open surgical applications, and minimally invasive applications such as laparoscopy.

The applicator 12 generally includes a support structure 18 having a body 20 and a handle 22 for a user to grip by hand. The handle 22 includes a gas supply conduit 24 for being operatively connected to a pressurized gas supply, which is not shown in the figures described herein. The handle 22 also supports a control unit 26 for operatively and selectively dispensing gas and reactive liquid from the tip 16. According to the exemplary embodiment, the control unit 26 includes a trigger 28 configured to be manipulated by a finger or fingers of the user while gripping the handle 22 by hand. The trigger 28 is biased from the handle 22 and, by applying force toward the handle 22, the user may selectively manipulate the trigger 28 toward the handle 22 for operating the applicator 12. While the trigger 28 of the control unit 26 is movable for selectively operating the applicator 12, it will be appreciated that any known method of controls for providing user input may be used for operating the applicator 12. For example, rather than moving the trigger 28, the control unit 26 may accept user input via one or more mechanical switches, electrical switches, or computer interfaces for dispensing the gas and the reactive liquid.

The body 20 is adapted to support a pair of vials 30a, 30b, or other suitable containers for holding liquid. Alternatively, the body 20 may support just a single container of liquid or more than two containers. Each vial 30a, 30b contains one of the reactive liquids for dispensing from the tip 16. A drive unit 32 is connected to the body 20. The drive unit 32 is operatively connected to the control unit 26 for selectively dispensing the liquid from the tip 16. According to the exemplary embodiment, the body 20 further includes a pair of vial housings 34a, 34b for receiving the pair of vials 30a, 30b. The vial housings 34a, 34b each include coupling ends 36a, 36b having liquid passages 38a, 38b. The coupling ends 36a, 36b each connect to the proximal ends 17a, 17b of the cannula 14 for fluidly communicating the liquids from the vials 30a, 30b, and into the cannula 14. The drive unit 32 selectively actuates and engages the vials 30a, 30b to create pressure within the vials 30a, 30b for discharging the liquid from the liquid passages 38a, 38b and into the proximal ends 17a, 17b. The liquids are maintained within separate lumens (not shown) while moving toward the distal end 17d for dispensing from the tip 16. The tip 16 includes outlets 39a, 39b in respective fluid communication with the proximal ends 17a, 17b. Thus, under pressure, liquid from the vial 30a dispenses from the outlet 39a, while the liquid from the vial 30b dispenses from the outlet 39b.

The drive unit 32 operatively creates pressure within the vials 30a, 30b for dispensing liquid from the outlets 39a, 39b at a predetermined rate of flow. More particularly, the drive unit 32 actuates the vials 30a, 30b according to the predetermined rate to maintain a consistent pressure within the vials 30a, 30b. Thus, regardless of the force with which the user selectively manipulates the trigger 28 to dispense the liquid from the tip 16, the liquid dispenses according to the predetermined rate. While the drive unit 32 applies force to the vials 30a, 30b for pressurizing the liquid, it will be appreciated that any container capable of holding liquid may be used, and, as such, any drive unit 32 able to apply sufficient pressure to force the liquid from the container may be so used. Additionally, the exemplary embodiment of the device 10 is configured to dispense two reactive liquids; however, it will be appreciated that the device 10 may be similarly configured for dispensing any number of liquids for use in the desired procedure.

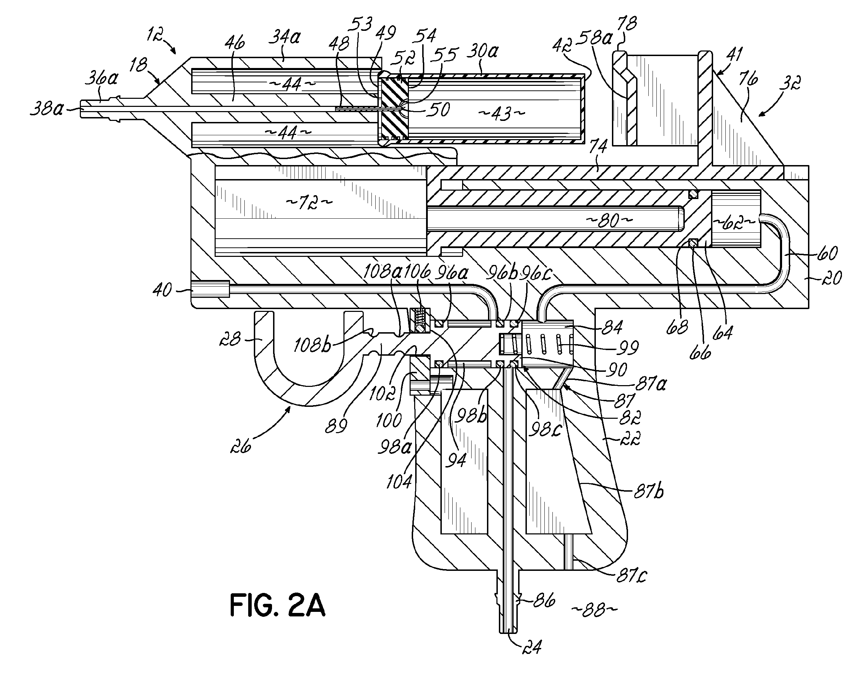

With respect to FIGS. 1 and 2A, the body 20 also includes a gas passage 40 in fluid communication with the gas supply end 17c of the cannula 14 for dispensing gas from the tip 16. The gas passage 40 is in selective fluid communication with the gas supply conduit 24 and operatively connected to the trigger 28. Accordingly, the user, via the trigger 28, selectively directs gas from the gas supply conduit 24 to the gas passage 40 for discharging gas from the applicator 12 and into the gas supply end 17c. The gas moves along the cannula 14 separated from the liquids while moving toward the distal end 17d. At the tip 16, the gas is dispensed from the tip 16 at both the outlets 39a, 39b in conjunction with the liquids. As such, the liquids mix and form into droplets under the influence of the pressurized gas. According to the exemplary embodiment, the support structure 18 is a unitary, handheld structure having the body 20 directly connected to the handle 22. However, it will be appreciated that the support structure 18 may, in the alternative, be formed of any number of independent components that are operatively connected together. For example, separate mechanical structures may each independently support the vials 30a, 30b, the drive unit 32, and the control unit 26 such that the device 10, rather than being a handheld unit, is a system of components capable of similarly dispensing the liquid and the gas from the tip 16.

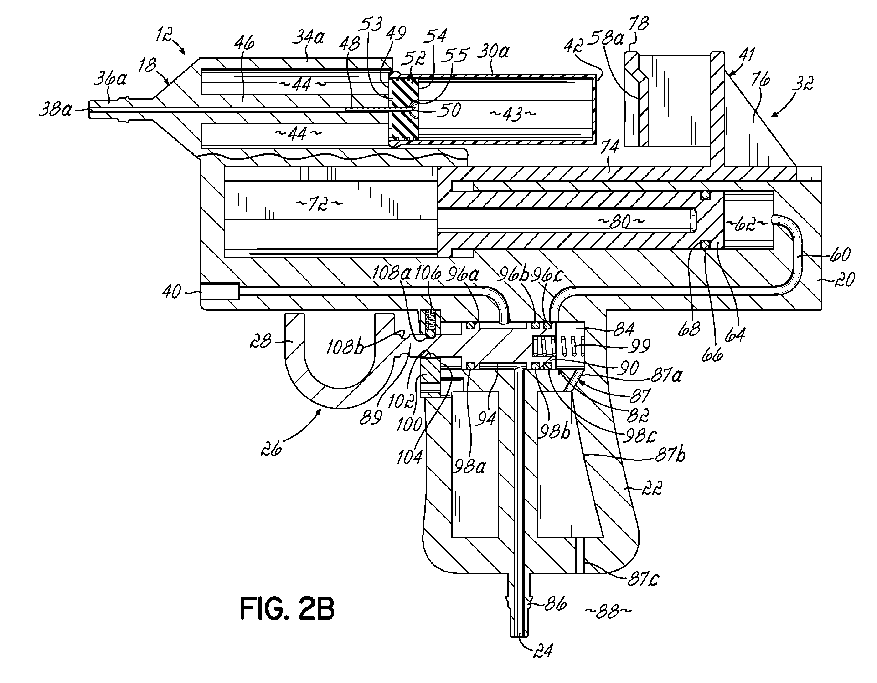

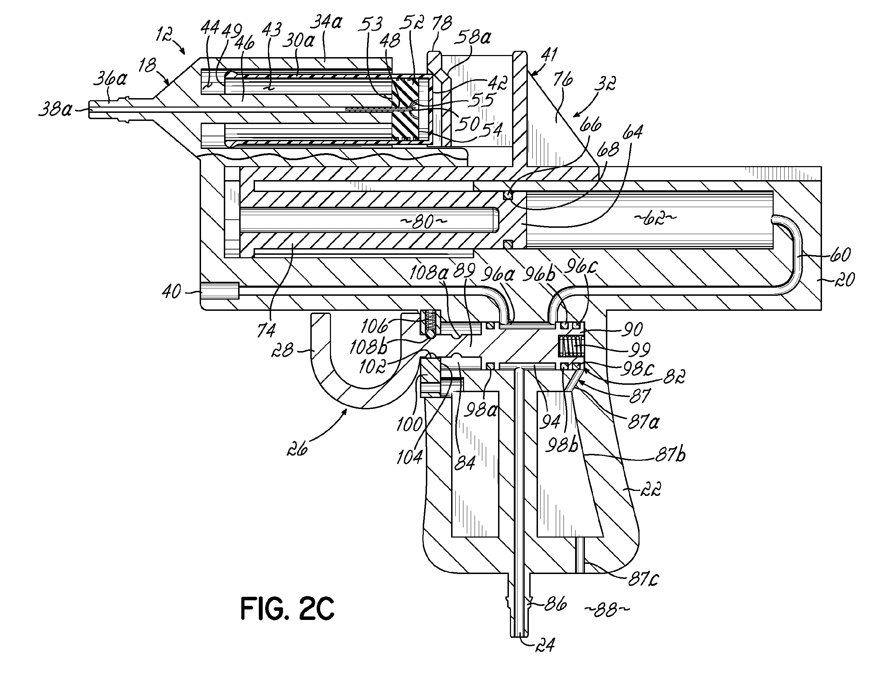

FIGS. 2A, 2B, and 2C are cross-sectional views taken generally along line 2-2 of FIG. 1 for showing the control unit 26, the drive unit 32, and the fluid connection of the applicator 12 with the vial 30a, which is similar to the fluid connection with vial 30b (see FIG. 1). The drive unit 32 has an actuator 41 that actuates to engage a rear end 42 of the vial 30a as directed by the control unit 26. Once the actuator 41 engages the vial 30a, the actuator 41 moves the vial 30a from right to left, as shown in FIG. 2C. The movement of the vial 30a forces liquid from within a liquid chamber 43 of the vial 30a and into the liquid passage 38a.

The vial housing 34a is generally sized to receive the vial 30a as the vial 30a moves from right to left. The vial housing 34a includes an annular void 44, a plunger 46, and the coupling end 36a. The plunger 46 extends from the coupling end 36a toward the vial 30a and within the annular void 44. A needle 48 is also connected to the plunger 46 extending toward a front end 49 of the vial 30a. Thus, the liquid passage 38a extends through the coupling end 36a, the plunger 46, and a lumen 50 within the needle 48. As the vial 30a moves, the needle 48 pierces a plug 52 positioned at the front end 49 within the liquid chamber 43 so that the liquid chamber 43 and the liquid passage 38a are fluidly connected.

The plug 52 includes a forward face 53 that engages the plunger 46 as the vial 30a moves from right to left. Because the plunger 46 is relatively stationary while the vial 30a is received by the annular void 44, the plug 52 contained therein forcibly slides within the vial 30a from the front end 49 toward the rear end 42. Thereby, the liquid chamber 43 gradually reduces in volume, which increases the pressure therein and expels the liquid from the vial 30a to discharge the liquid from the liquid passage 38a according to the predetermined flow rate. In addition, the plug 52 includes a rear face 54 having a needle recess 55 for receiving the needle 48. More particularly, the needle 48 pierces the plug 52 such that the needle terminates within the needle recess 55 prior to moving beyond the rear face 54. In this respect, the vial 30a may move from right to left until the rear end 42 of the vial 30a contacts the rear face 54 of the plug 52 without directly contacting the needle 48. This cooperation both prevents damage between the needle 48 and the vial 30 while also allowing for further reduction in volume of the liquid chamber 43 for expelling the liquid.

The vial 30a is selectively actuated by the drive unit 32 in order to selectively discharge the liquid. Generally, any drive unit 32 capable of actuating the vial 30a may be used. For instance, the drive unit 32 may be mechanical, electronic, pneumatic, or any combination thereof for imparting movement to the vial 30a. According to the exemplary embodiment, the drive unit 32 is a pneumatic drive unit 32 having the actuator 41 selectively operable to actuate the vial 30a. More particularly, the actuator 41 is operatively connected to the control unit 26 and translates toward the plunger 46 until the rear end 42 of the vial 30a is received within a recess 58a of the actuator 41, which improves the stability of the vial 30a supported by the body 20. Each vial 30a, 30b (see FIG. 1) is received by respective recesses 58a, 58b (see FIG. 1).

As the vial 30a actuates, the liquid discharges from the liquid passage 38a according to the predetermined rate of flow. However, the predetermined rate of flow may be selectively variable. First, the pressurized gas supply, which operatively actuates the actuator 41, generally is preselected at a pressure above zero pounds per square inch (psi) and below 40 psi. Particularly, the pressurized gas supply may be between 10 psi and 30 psi. More particularly, the exemplary embodiment of the pressurized gas supply is preselected at a pressure of generally 20 psi. However, the pressurized gas supply may be preselected at any pressure to select the predetermined rate of flow from the vial 30a. Second, the needle 48 has a lumen 50 that is sized to limit the flow from the vial 30a according to the predetermined rate of flow. However, the needle 48 is removably connected to the plunger 46. Thus, in order to vary the predetermined rate of flow for particular application or fluids, the needle 48 with the lumen 50 may be exchanged for another needle (not shown) having a lumen sized to limit flow according to another predetermined rate. According to the exemplary embodiment, the needle 48 is an approximately 19 gauge, thin wall, hypo-tube needle. While the pressurized gas supply and removable needle 48 may be independently used to achieve the predetermined rate of flow, it will be appreciated that any combination thereof may be used to achieve the predetermined rate for various applications and/or fluids. Such fluids may include, but are not limited to biomaterials, such as fibrin and thrombin. In the case where the fluids are fibrin and thrombin, the gas supply pressure is generally 20 psi, and the needle 48 is a generally 19 gauge, thin wall, hypo-tube needle, the predetermined rate of fluid flow from both vials 30a, 30b (see FIG. 1), cumulatively, is between approximately 5 milliliters per minute and approximately 10 milliliters per minute.

As discussed briefly above, the pneumatic drive unit 32 drives the actuator 41 under the influence of pressurized gas selectively delivered to the pneumatic drive unit 32 via a gas conduit 60. The pressurized gas is maintained at constant pressure in order to achieve the predetermined flow rate. However, as described above, the user may vary the pressure of the pressurized gas supply in order to vary the predetermined flow rate. The exemplary embodiment of the pneumatic drive unit 32 further includes a cylinder 62 and a piston 64. The piston 64 is connected to the actuator 41 and slidably inserted into the cylinder 62. Furthermore, the piston 64 includes a piston o-ring 66 on an annular piston groove 68 sized to operatively seal the cylinder 62 so that pressurized gas may be held within the cylinder 62. The cylinder 62 is fluidly connected to the gas conduit 60 for receiving the pressurized gas. The gas conduit 60 is operatively connected to the pneumatic control unit 26 for selectively powering the pneumatic drive unit 32, which, in the exemplary embodiment, fluidly connects the cylinder 62 to the gas supply conduit 24.

As the cylinder 62 is pressurized, the piston 64 slides or otherwise advances from a rearward position within the cylinder 62, shown in FIG. 2A, toward a forward position (see FIG. 2C) according to the predetermined rate. The body 20 also includes a cavity 72 adapted to provide clearance for the advancing piston 64. The piston 64 is operatively connected to the actuator 41 for transmitting the movement of the piston 64 to the actuator 41. According to the exemplary embodiment of the invention, the actuator 41 includes a connecting member 74, a bracing member 76, and a forcing member 78 having the recess 58a. The connecting member 74 is connected to the piston 64 and extends rearward to the bracing member 76, which is connected thereto. The bracing member 76 is, in turn, connected to the forcing member 78, which extends forward of the bracing member 76. Generally, the bracing member 76 is adapted to provide structural support for the forcing member 78 while the pneumatic drive unit 32 forces the actuator 41 against the vials 30a, 30b (see FIG. 1). It will be appreciated that the actuator 41 may be connected to the piston 64 in various structural configurations for moving the vials 30a, 30b according to the predetermined rate, as shown in FIG. 1. However, the present embodiment of the invention having the connecting member 74 extending rearwardly from the piston 64 provides for a generally compact, handheld configuration of the pneumatic drive unit 32. Similarly, the piston 64 may also include a bore 80 extending therethrough for reducing the weight of the pneumatic drive unit 32.

The body 20 also includes the gas passage 40 that is selectively connected to the gas supply conduit 24. The gas passage 40 is adapted for being connected to the cannula 14 (see FIG. 1) for selectively dispensing gas from the tip 16. Also, as briefly described above, the control unit 26 is operatively connected to the gas passage 40 to selectively and fluidly connect the gas passage 40 to the gas supply conduit 24. Accordingly, the gas supply at the gas supply conduit 24 for operating the pneumatic control unit 26 is the same gas supply connected to the gas passage 40. However, it will be appreciated that the control unit 26 may selectively connect the gas passage 40 to another gas supply (not shown) for discharging gas from the gas passage 40. In other words, the gas discharged from the gas passage 40 may be a first gas, while the gas operatively connected to the pneumatic drive unit 32 may be a second gas which is the same or different type of gas from a different supply. It will be further appreciated that any type of pressurized gas may be used for operating the pneumatic drive unit 32 and/or discharging from the gas passage 40. Such gases include, but are not limited to, nitrogen, carbon dioxide, air, or any other gas having properties compatible for use during the desired procedure.

The handle 22 is connected to the body 20 and extends generally perpendicularly therefrom. The handle 22 supports the control unit 26, which may be adapted for use by the user while gripping the handle 22 by hand. As briefly described above, the control unit 26, based on input from the user, selectively discharges gas from the gas passage 40 and selectively discharges liquid from the liquid passages 38a, 38b (see FIG. 1). More particularly, the control unit 26 includes the trigger 28 for selectively directing the control unit 26 to discharge the gas and/or liquid. While the trigger 28 may be used according to the exemplary embodiment for selectively discharging gas and/or liquid from the applicator 12, it will be appreciated that any form of user controls, such as electrical or electronic, mechanical, pneumatic, or any combination thereof may be used to dispense the gas and/or liquid from the tip 16.

The single control unit 26, having the trigger 28, discharges the gas and/or the liquid according to three modes of operation. During a first mode, the control unit 26 does not actuate the pneumatic drive unit 32 and does not fluidly connect the gas supply conduit 24 to the gas passage 40. Neither liquid nor gas is discharged from the applicator 12 during the first mode, which may otherwise be referred to as simply an off mode. During a second mode, the control unit 26 fluidly only connects the gas supply conduit 24 to the gas passage 40 such that only gas is discharged from the applicator 12. During a third mode, the control unit 26 simultaneously actuates the pneumatic drive unit 32 and fluidly connects the gas supply conduit 24 to the gas passage 40. Accordingly, both gas and liquid are simultaneously discharged from the applicator 12 during the third mode.

The user may selectively direct the control unit 26 to discharge gas and/or liquid according to the first, second, or third modes. However, the control unit 26 must selectively operate the first, second, and third modes in a "sequential" manner. As used herein, the term "sequential" means that the control unit 26 operates in sequence when selecting the modes according to forward or reverse sequential orders of the first, second, third modes. For instance, the user may selectively direct the control unit 26 from the first mode to the second mode. From the second mode, the user may selectively direct the control unit 26 to either the first mode or the third mode. From the third mode, the user may selectively direct the control unit 26 to the second mode. However, the user may not selectively direct the control unit 26 directly from the first mode to the third mode, or vice versa, without having directed the control unit 26 into to the second mode. In essence, the sequential manner of the control unit 26 prevents the user from bypassing the second mode during use.

Such restrictions on the control unit 26 promote preferred application of the gas and the liquid during medical procedures. For example, the gas discharged from the applicator 12 in the second mode is used to dry or otherwise prepare a surface (not shown) for application of the liquid. Additionally, the gas discharged from the applicator 12 in the second mode is used to clean or otherwise remove the liquid from the tip 16. Such cleaning improves the useful life of the tip 16 and prevents the reactive liquids from curing or otherwise clogging the tip 16 that may remain within the tip 16 following the third mode. In order to provide both drying and cleaning benefits, the first, second, and third modes are limited such that the second mode must occur before and after the third mode in operation.

The control unit 26 may be selectively and operatively connected to the gas passage 40 and the pneumatic drive unit 32 by mechanical controls, electrical or electronic controls, pneumatic controls, or any combination thereof for dispensing the gas and the liquid according to the three modes described above. However, according to the exemplary embodiment of the invention, the control unit 26 is a pneumatic control unit 26 having a valve 82 fluidly connected to the gas supply conduit 24, the gas passage 40, and the pneumatic drive unit 32 for selectively directing the gas to operate the applicator 12.

The pneumatic control unit 26 includes a valve chamber 84. The valve chamber 84 is formed within the handle 22 for supporting the pneumatic control unit 26; however, it will be appreciated that the valve chamber 84, in the alternative, may be directly or indirectly supported by the support structure 18. For example, the pneumatic control unit 26 may, in the alternative, have its own housing (not shown) defining the valve chamber 84. In any case, the valve chamber 84 is in fluid communication with the gas supply conduit 24, gas passage 40, and the pneumatic drive unit 32 via the gas conduit 60. The handle 22 includes a gas connector 86 having the gas supply conduit 24 extending therethrough. From the gas connector 86, the gas supply conduit 24 extends to fluidly connect with the valve chamber 84. In addition, the gas passage 40 and the gas conduit 60 each extend through the body 20 and toward the handle 22 until fluidly connecting with the valve chamber 84. The handle 22 also includes a vent passage 87 extending from the valve chamber 84 to an exterior environment 88 open to atmospheric pressure. The vent passage 87 fluidly connects the valve chamber 84 to the exterior environment 88 for relieving pressure, or excess gas, within the cylinder 62 of the pneumatic drive unit 32. For example, the cylinder 62 may receive pressurized gas to actuate the vials 30a, 30b (see FIG.1) by driving the piston 64. Once the cylinder 62 no longer receives pressurized gas, the cylinder 62 continues to retain the remaining pressure until that pressure is relieved. Without the vent passage 87, the remaining pressure caused by excess gas would continue to move the piston 64 until sufficiently diminished, which needlessly dispenses reactive liquid. With the vent passage 87, the remaining pressure in the cylinder 62 vents to the atmosphere so that the piston 64 stops moving once the pressurized gas is disconnected from the cylinder 62. In this way, the vial 30a either actuates at the predetermined rate or stops actuating altogether depending on the position of the valve 82 described below in more detail.

The vent passage 87 of the exemplary embodiment includes a first vent passage portion 87a, a second vent passage portion 87b, and a third vent passage portion 87c. The first vent passage portion 87a is fluidly connected between the valve chamber 84 and the second vent passage portion 87b. The second vent passage portion 87b is in the form of a void within the handle 22 and extends from the first vent passage portion 87a to the third vent passage portion 87c. The third vent passage portion 87c is in fluid communication with the exterior environment 88 at atmospheric pressure. Thus, the remaining pressure within the cylinder 62 may vent through the vent passage 87. However, it will be appreciated that other channels or passages may also be formed in order to vent pressure from cylinder 62.

The pneumatic control unit 26 further includes the trigger 28, as briefly described above, a stem member 89, and a spool valve 90. The trigger 28 is connected to the stem member 89, which extends through the body 20 and into the valve chamber 84. The spool valve 90 is also positioned within the valve chamber 84 and movable or otherwise slidable within the valve chamber 84. The stem member 89 is connected to the spool valve 90 such that the user manipulating the trigger 28 may selectively move the spool valve 90 along the valve chamber 84. More particularly, the spool valve 90 is selectively movable in a sequential manner to first, second, and third positions within the valve chamber 84 for selectively connecting the gas supply conduit 24 to one or both of the gas passage 40 and the gas conduit 60. The first, second, and third positions correspond to the first, second, and third modes of operation, respectively. Thus, the spool valve 90 moves from one position to another in the sequential manner described above. Essentially, the spool valve 90 moves between the first and third positions by way of the second position for preventing the user from bypassing the second position during use as discussed above.

The spool valve 90 further includes an annular passage 94 and first, second, and third annular valve grooves 96a, 96b, 96c with respective first, second, and third o-rings 98a, 98b, 98c. Generally, the first and second annular valve grooves 96a, 96b, having the first and second o-rings 98a, 98b positioned therein, bound the annular passage 94 for operatively sealing the annular passage 94 from the valve chamber 84. In this way, the annular passage 94 may be moved along the valve chamber 84 to selectively connect the gas supply conduit 24 to one or both of the gas passage 40 and the gas conduit 60 for directing pressurized gas without leaking pressurized gas freely into the valve chamber 84.

The pneumatic control unit 26 also includes a first biasing element 99 for biasing the spool valve 90 in the first position as shown in FIG. 2A. According to the exemplary embodiment, the first biasing element 99 is a spring. The first biasing element 99 is positioned against the spool valve 90 such that the spool valve 90 is resiliently mounted within the valve chamber 84. The spool valve 90 is located in the first position to cover the gas supply conduit 24 leading into the valve chamber 84 and seal the gas supply conduit 24 from both the gas passage 40 and the gas conduit 60. As such, the second o-ring 98b and the third o-ring 98c are positioned between the gas supply conduit 24 and the gas passage 40, respectively, to operatively seal each from the gas supply conduit 24. In addition, with the spool valve 90 in the first position, the first vent passage portion 87a is positioned such that the gas conduit 60 and the first vent passage portion 87a are in fluid communication for relieving pressure from within the cylinder 62. However, the spool valve 90 also blocks the gas supply conduit 24 from fluid communication with the first vent passage 87a in order to prevent the leakage of supplied pressurized gas to the exterior environment 88.

The support structure 18 further includes a collar 100 positioned at an opening 102 of the valve chamber 84. The collar 100 generally surrounds the stem member 89 for further supporting the stem member 89 and providing a stop surface 104 against which the first biasing element 99 biases the spool valve 90. Generally, the spool valve 90 positioned against the stop surface 104 is indicative of the first position shown in FIG. 2A. However, the collar 100 may also include a second biasing element 106 adapted to snap into first and second detents 108a, 108b located along the stem member 89. More particularly, the first and second detents 108a, 108b are configured to be positioned along the stem member 89 to be indicative of the second position and the third position, respectively. Thereby, the snapping of the second biasing element 106 into the first and second detents 108a, 108b notifies the user selectively manipulating the trigger 28 of the position of the spool valve 90.

FIG. 2B shows the spool valve 90 in the second position for fluidly connecting only the gas supply conduit 24 to the gas passage 40 via the annular passage 94. Accordingly, the second biasing element 106 is snapped into the first detent 108a. Specifically, the spool valve 90 is retracted from the first position such that the annular passage 94 fluidly connects the gas supply conduit 24 to the gas passage 40. However, in the second position, the second o-ring 98b continues to seal the gas supply conduit 24 from the gas conduit 60. Thus, the gas supply conduit 24 remains fluidly disconnected from the pneumatic control unit 26. According to the exemplary embodiment of the invention, the user may selectively pass through the second mode via the pneumatic control unit 26 without regard to the amount of time spent dispensing gas according to the second mode. In the alternative, the pneumatic control unit 26 may be adapted to remain fixed in the second mode for a predetermined minimum amount of time. The predetermined amount of time may also be optimized for cleaning the liquid from the tip 16 and/or preparing the anatomical site for the medical procedure. With respect to the vent passage 87 with the spool valve 90 in the second position, the gas conduit 60 is still in fluid communication with the first vent passage portion 87a through the valve chamber 84. However, the position of the spool valve 90 with respect to the gas conduit 60 shown in FIG. 2B is slightly exaggerated to emphasize the fluid connection therebetween. According to an exemplary embodiment, the o-ring 98c is centered where the gas conduit 60 connects to the valve chamber 84 so that pressurized gas may flow around the o-ring 96c and into the first vent passage portion 87a. In any case, the remaining pressure within cylinder 62 is also relieved while the spool valve 90 is in the second position.

FIG. 2C shows the spool valve 90 in the third position for fluidly connecting both the gas passage 40 and the gas conduit 60 to the gas supply conduit 24 via the annular passage 94. Accordingly, the second biasing element 106 is snapped into the second detent 108b. Specifically, the spool valve 90 is retracted further from the second position such that the annular passage 94 fluidly connects the gas supply conduit 24 simultaneously to the gas passage 40 and the gas conduit 60. As such, in the third position, the second and third o-rings 98b, 98c seal the valve chamber 84 against the annular passage 94 from the gas conduit 60 to prevent pressurized gas from leaking into the remainder of the valve chamber 84. With respect to the vent passage 87 with the spool valve 90 in the third position, the spool valve 90 blocks the gas conduit 60 from fluidly communicating with the first vent passage portion 87a. In addition, the first vent passage portion 87a is also blocked by the spool valve 90. Thus, pressurized gas may be delivered along the gas conduit 60 and into the cylinder 62 without leaking pressurized gas into the first vent passage portion 87a.

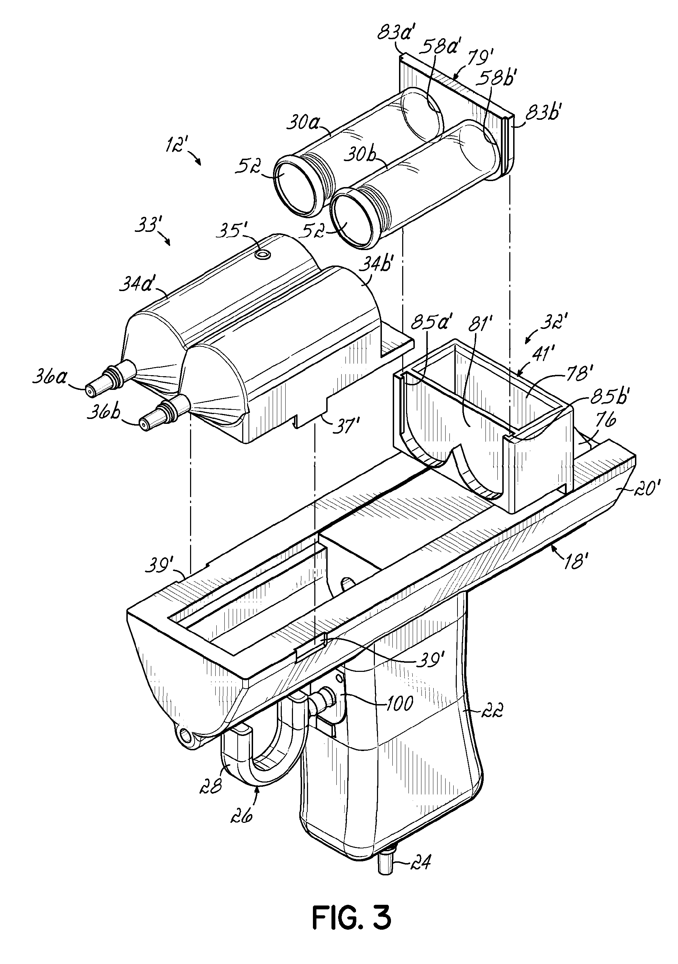

With respect to FIG. 3, an alternative embodiment of an applicator 12' for dispensing a reactive liquid and a gas includes a modular vial receiver 33' having a pair of vial housings 34a', 34b'. As such, like numbers shown in FIG. 3 indicate like features of the applicator 12 (see FIG. 1). The modular vial receiver 33' also includes a pair of clips 37' adjacent to each of the vial housings 34a', 34b' for removably attaching the modular vial receiver 33' to a body 20' of a support structure 18'. More particularly, the body 20' includes a pair of grooves 39' adapted to cooperate with the respective clips 37' for attaching and/or detaching the modular vial receiver 33' to the body 20'. Each the clip 37' extends toward the respective groove 39' and is received by and "snapped" into the groove 39'.

Furthermore, the applicator 12' includes a drive unit 32'. The drive unit 32' includes an actuator 41' with a forcing member 78'. The forcing member 78' includes a removable vial key member 79'. The vial key member 79' includes a pair of recesses 58a' and 58b' for receiving the vials 30a, 30b respectively and ensuring that the vials 30a, 30b are installed in the applicator 12' correctly. More particularly, the vials 30a, 30b may be attached to the vial key member 79' within the recesses 58a', 58b'. The forcing member 78' also includes a key recess 81' that opposes the modular vial receiver 33'. The key recess 81' is configured to removably receive the vial key member 79' therein for positioning the vials 30a, 30b adjacent to the vial housings 34a', 34b' as described above with respect to the applicator 12 (see FIG. 1). Proper installation of the vials 30a, 30b, may also be indicated by color coding the plug 52 in each of the vials 30a, 30b. For example, the plug 52 of the vial 30a may be a first color and the plug 52 of the vial 30b may be a second color. As such, the first and second colors may be indicative of the contents of each vial 30a, 30b for improving installation. Further indication may be positioned on one or both of the vial housings 34a', 34b' for coordinating the vials 30a, 30b for installation. For example, the vial housing 34a includes an indicia 35' for indicating that vial 30a should be received by vial housing 34a'. However, it will be appreciated that other indicia may be used for indicating proper installation of the vials 30a, 30b with vial housing 34a', 34b'.

The vial key member 79' includes a pair of lip projections 83a', 83b' that cooperate with a pair of channels 85a', 85b' adjacent to the key recess 81'. Specifically, the pair of lip projections 83a', 83b' slide into the pair of channels 85a', 85b' respectively such that the vial key member 79' is positioned within the key recess 81'.

During use, the modular vial receiver 33' is attached to the body 20' by snapping the clips 37' into the grooves 39'. Also, the removable key member 79' engages the remainder of the forcing member 78' by sliding the lip projections 83a', 83b' into the respective channels 85a', 85b'. Accordingly, the vials 30a, 30b are attached to the vial key member 79' such that the vial key member 79' will only engage the key recess 81' if installed properly. In this way, the cooperating vial key member 79' and the key recess 81' ensure that the vial housing 34a' receives the vial 30a and that the vial housing 34b' receives the vial 30b.

The applicator 12' discharges and dispenses liquid from the vials 30a, 30b as described above. While discharging and dispensing the liquid, the modular vial receiver 33' and the vials 30a, 30b likely contact the liquid. Thus, rather than discard the entire applicator 12 (see FIG. 1) after use, the modular vial receiver 33', the vials 30a, 30b, and the vial key member 79' may be removed from the body 20' and the remainder of the forcing member 78', respectively. Once removed from the remainder of applicator 12', the modular vial receiver 33', the vials 30a, 30b, and the attached vial key member 79' may each be replaced with a clean modular vial receiver 33' and new vials 30a, 30b of liquid attached to a new vial key member 79'. Finally, the replaced modular vial receiver 33', vials 30a, 30b, and vial key member 79' are discarded for disposal. However, in the alternative, the replaced modular vial receiver 33', vials 30a, 30b, and vial key member 79' may be cleaned and reused if practical.

A device and method for dispensing a liquid and a gas from a tip includes a support structure, a control unit, and a drive unit. The liquid is held within a container. The support structure is adapted to hold the container. The control unit directs the drive unit to expel liquid from the container according to a predetermined rate. The control unit further directs the drive unit according to first, second, and third modes of operation. During the first mode, neither gas nor liquid is dispensed from the device. During the second mode, only gas is dispensed from the device. During the third mode, both gas and liquid are dispensed from the device. Furthermore, the control unit is only selectable and operable by a user through the first, second, and third modes in a sequential manner.

**The invention is further described by the following embodiments, wherein:**
Embodiment 1. A device for dispensing a liquid and a gas from a tip, the liquid held within a container, comprising;
   a support structure adapted for holding the container, the support structure having a liquid passage, a gas passage, and a gas supply conduit, the liquid passage adapted to communicate with the container, the gas supply conduit configured to be in selective fluid communication with the gas passage for selectively discharging the gas from the gas passage;
   a drive unit connected to the support structure, the drive unit configured to actuate for selectively expelling the liquid from the container and selectively discharging the liquid from the liquid passage; and
   a control unit connected to the support structure, the control unit operatively connected to the drive unit and the gas supply conduit and operative to allow selection and operation according to a first, second, and third mode of operation, the first mode neither actuating the drive unit nor fluidly connecting the gas supply conduit to the gas passage, the second mode fluidly only connecting the gas supply conduit to the gas passage, and the third mode actuating the drive unit and fluidly connecting the gas supply conduit to the gas passage,
   wherein the control unit is operable by a user such that the first, second, and third modes can only be selected and operated in a sequential manner.
Embodiment 2. The device with the features of embodiment 1 further including a cannula in fluid communication with the liquid passage and the gas passage, the cannula having a distal end and a proximal end, the proximal end connected to the liquid passage, the distal end having the tip attached thereto, the tip configured for dispensing the liquid and the gas.
Embodiment 3. The device with the features of embodiment 1 wherein the drive unit is adapted to discharge the liquid from the liquid passage at a predetermined rate.
Embodiment 4. The device with the features of embodiment 1 wherein the support structure is a unitary support structure.
Embodiment 5. The device with the features of embodiment 1 wherein the control unit is adapted to remain in the second mode for at least a predetermined minimum amount of time.
Embodiment 6. A device for dispensing a liquid and a gas from a tip for use in a medical procedure, the liquid held within a container, comprising;
   a support structure adapted for holding the container, the support structure having a liquid passage, a gas passage, and a gas supply conduit, the liquid passage adapted to communicate with the container, the gas supply conduit configured to be in selective fluid communication with the gas passage for selectively discharging the gas from the gas passage;
   a pneumatic drive unit connected to the support structure and in selective fluid communication with the gas supply conduit, the pneumatic drive unit configured to actuate via the gas for selectively expelling the liquid from the container and selectively discharging the liquid from the liquid passage; and
   a valve connected to the support structure and in fluid communication with the gas supply conduit, gas passage, and the pneumatic drive unit, the valve being movable between first, second, and third positions, the first position fluidly disconnecting the pneumatic drive unit from the gas supply conduit, the second position fluidly only connecting the gas passage to the gas supply conduit, and the third position fluidly connecting both the pneumatic drive unit and the gas passage to the gas supply conduit,
   wherein the valve is operable by a user such that the first, second, and third positions can only be selected and operated in a sequential manner.
Embodiment 7. The device with the features of embodiment 6 further including a cannula in fluid communication with the liquid passage and the gas passage, the cannula having a distal end and a proximal end, the proximal end connected to the liquid passage, the distal end having the tip attached thereto, the tip configured for dispensing the liquid and the gas according to the medical procedure.
Embodiment 8. The device with the features of embodiment 6 wherein the valve further comprises:
   a valve chamber in fluid communication with the gas supply conduit, the gas passage, and the pneumatic drive unit, the chamber having a longitudinal axis,
   a spool valve positioned within the chamber, the spool valve being slidable between the first, second, and third positions along the longitudinal axis.
Embodiment 9. The device with the features of embodiment 8 wherein the spool valve includes an annular passage, the annular passage adapted to selectively communicate gas through the valve chamber.
Embodiment 10. The device with the features of embodiment 8 wherein the pneumatic control unit is biased in the first position.
Embodiment 11. The device with the features of embodiment 8 wherein the spool valve is resiliently mounted within the valve chamber.
Embodiment 12. The device with the features of embodiment 11 wherein the control unit has a trigger adapted for being manipulated by a user for selectively moving the valve.
Embodiment 13. The device with the features of embodiment 6 wherein the support structure includes a collar having a stop surface, the collar supporting the valve resiliently mounted within the valve chamber, the valve being biased against the stop surface in the first position.
Embodiment 14. The device with the features of embodiment 13 wherein the collar further includes a second biasing element and the valve further includes a detent, the second biasing element adapted to snap into the detent when the valve is positioned in a second or third position.
Embodiment 15. The device with the features of embodiment 6 wherein the pneumatic drive unit is adapted to discharge the liquid from the liquid passage at a constant rate.
Embodiment 16. The device with the features of embodiment 6 wherein the support structure is a unitary support structure.
Embodiment 17. The device with the features of embodiment 6 wherein the control unit is adapted to position the valve in the second position for at least a predetermined minimum amount of time.
Embodiment 18. The device with the features of embodiment 6 further comprising a vent passage extending from the valve to an exterior environment, the first and second positions of the valve fluidly connecting the vent passage to the pneumatic drive unit for relieving excess gas from the pneumatic drive unit to the exterior environment, the third position of the valve fluidly disconnecting the pneumatic drive unit from the valve passage.
Embodiment 19. A device for dispensing a liquid and a gas from a tip, the liquid held within a container having a plug inserted therein, comprising;
   a support structure adapted for holding the container, the support structure having a liquid passage, a gas passage, and a gas supply conduit, the liquid passage adapted to communicate with the container, the gas supply conduit configured to be in selective fluid communication with the gas passage for selectively discharging the gas from the gas passage;
   a drive unit connected to the support structure, the drive unit configured to actuate for selectively expelling the liquid from the container and selectively discharging the liquid from the liquid passage;
   a control unit connected to the support structure, the control unit operatively connected to the drive unit to selectively actuate the drive unit, the control unit operatively connected to the gas supply conduit to selectively fluidly connect the gas supply conduit to the gas passage;
   wherein, the control unit directs the drive unit to expel liquid according to a predetermined rate.
Embodiment 20. The device with the features of embodiment 19 further including a cannula in fluid communication with the liquid passage and the gas passage, the cannula having a distal end and a proximal end, the proximal end connected to the liquid passage, the distal end having the tip attached thereto, the tip configured for dispensing the liquid and the gas.
Embodiment 21. The device with the features of embodiment 19 wherein the predetermined rate is variable.
Embodiment 22. The device with the features of embodiment 19 wherein the support structure is a unitary support structure.
Embodiment 23. The device with the features of embodiment 19 wherein the control unit has a trigger adapted for being manipulated by a user for selectively directing the drive unit.
Embodiment 24. The device with the features of embodiment 19 wherein the drive unit is a pneumatic drive unit.
Embodiment 25. The device with the features of embodiment 24 wherein the pneumatic drive unit further comprises a cylinder in selective fluid communication with the gas supply conduit; a piston positioned within the cylinder, the piston adapted to slide along cylinder; and an actuator operatively connected to the piston for actuating the container, wherein gas from the gas supply conduit is adapted to actuate the container according to the predetermined rate.
Embodiment 26. The device with the features of embodiment 25 further comprising a vent passage in fluid communication with the cylinder and an exterior environment, the vent passage adapted to selectively vent excess gas from the cylinder to the exterior environment so that the container actuates according to the predetermined rate.
Embodiment 27. The device with the features of embodiment 19 wherein the support structure further comprises a plunger having the liquid passage extending therethrough, the plunger adapted for being inserted into the container, wherein the drive unit is configured to actuate the plug against the plunger for forcing the plug into the container and expelling liquid into the liquid passage.
Embodiment 28. The device with the features of embodiment 27 further comprising a needle, the needle being connected to the plunger and having a lumen extending therethrough, wherein the needle is adapted for extending through the plug to fluidly connect the liquid passage to the container via the lumen.
Embodiment 29. A method of dispensing a liquid and a gas from a tip for use in a medical procedure, the liquid held within a container, comprising the steps of;
   selectively manipulating a single control unit to dispense only gas from the tip according to a second mode of operation;
   selectively manipulating the single control unit to simultaneously dispense gas and liquid from the tip according to a third mode of operation; and
   limiting the selective manipulation of the single control unit such that the second mode must occur before the third mode.
Embodiment 30. The method with the features of embodiment 29 further comprising:
   positioning a valve in a first position during the first mode, the first position fluidly disconnecting a gas supply conduit from a pneumatic drive unit and a gas passage;
   positioning the valve in a second position during the second mode, the second position fluidly connecting the gas supply conduit only to the gas passage;
   positioning the valve in a third position during the third mode, the third position fluidly connecting the gas supply conduit to the gas passage and the pneumatic drive unit; and
   moving the valve only between first and third positions by way of the second position.
Embodiment 31. The method with the features of embodiment 30 further comprising venting excess gas from the pneumatic drive unit to an exterior environment when the valve is in the first and second positions.
Embodiment 32. The method with the features of embodiment 29 further including biasing the valve in the first position.
Embodiment 33. The method with the features of embodiment 29 wherein the control unit is a pneumatic control unit.
Embodiment 34. A method of dispensing a liquid and a gas from a tip for use in a medical procedure, the liquid held within a container, comprising;
   selectively manipulating a control unit to simultaneously dispense the liquid and the gas from the tip; and
   dispensing the liquid according to a predetermined rate regardless of the manner in which the control unit is selectively manipulated.

While the present invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. The invention in its broader aspects is therefore not limited to the specific details, representative apparatus and method and illustrative examples shown and described. Accordingly, departures may be from such details without departing from the scope or spirit of the general inventive concept. What is claimed is:

## Claims

1. A device for dispensing a liquid and a gas from a tip, the liquid held within a container, comprising;
a support structure adapted for holding the container, the support structure having a liquid passage, a gas passage, and a gas supply conduit, the liquid passage adapted to communicate with the container, the gas supply conduit configured to be in selective fluid communication with the gas passage for selectively discharging the gas from the gas passage;
a drive unit connected to the support structure, the drive unit configured to actuate for selectively expelling the liquid from the container and selectively discharging the liquid from the liquid passage;
a control unit connected to the support structure, the control unit operatively connected to the drive unit to selectively actuate the drive unit, the control unit operatively connected to the gas supply conduit to selectively fluidly connect the gas supply conduit to the gas passage;
wherein, the control unit directs the drive unit to expel liquid according to a predetermined rate.

2. The device of claim 1, comprising;
the control unit operative to allow selection and operation according to a first, second, and third mode of operation, the first mode neither actuating the drive unit nor fluidly connecting the gas supply conduit to the gas passage, the second mode fluidly only connecting the gas supply conduit to the gas passage, and the third mode actuating the drive unit and fluidly connecting the gas supply conduit to the gas passage; and
wherein the control unit is operable by a user such that the first, second, and third modes can only be selected and operated in a sequential manner.

3. The device of claim 1, wherein the drive unit is a pneumatic drive unit which is in selective fluid communication with the gas supply conduit, the pneumatic drive unit configured to actuate via the gas for selectively expelling the liquid from the container and selectively discharging the liquid from the liquid passage; and
a valve connected to the support structure and in fluid communication with the gas supply conduit, gas passage, and the pneumatic drive unit, the valve being movable between first, second, and third positions, the first position fluidly disconnecting the pneumatic drive unit from the gas supply conduit, the second position fluidly only connecting the gas passage to the gas supply conduit, and the third position fluidly connecting both the pneumatic drive unit and the gas passage to the gas supply conduit,
wherein the valve is operable by a user such that the first, second, and third positions can only be selected and operated in a sequential manner.

4. The device of claims 1 through 3 further including a cannula in fluid communication with the liquid passage and the gas passage, the cannula having a distal end and a proximal end, the proximal end connected to the liquid passage, the distal end having the tip attached thereto, the tip configured for dispensing the liquid and the gas.

5. The device of claims 1 or 3 wherein the support structure is a unitary support structure.

6. The device of claims 2 or 3 wherein the control unit has a trigger adapted for being manipulated by a user for selectively directing the drive unit.

7. The device of claim 3 wherein the valve further comprises:
a valve chamber in fluid communication with the gas supply conduit, the gas passage, and the pneumatic drive unit, the chamber having a longitudinal axis,
a spool valve positioned within the chamber, the spool valve being slidable between the first, second, and third positions along the longitudinal axis.

8. The device of claim 7 wherein the spool valve:
a) includes an annular passage, the annular passage adapted to selectively communicate gas through the valve chamber; or
b) is resiliently mounted within the valve chamber.

9. The device of claims 1 through 3 wherein the support structure includes a collar having a stop surface, the collar supporting the valve resiliently mounted within the valve chamber, the valve being biased against the stop surface in the first position.

10. The device of claim 3 wherein the pneumatic drive unit further comprises a cylinder in selective fluid communication with the gas supply conduit; a piston positioned within the cylinder, the piston adapted to slide along cylinder; and an actuator operatively connected to the piston for actuating the container, wherein gas from the gas supply conduit is adapted to actuate the container according to the predetermined rate.

11. The device of claim 10 further comprising a vent passage in fluid communication with the cylinder and an exterior environment, the vent passage adapted to selectively vent excess gas from the cylinder to the exterior environment so that the container actuates according to the predetermined rate.

12. The device of claim 3 wherein the support structure further comprises a plunger having the liquid passage extending therethrough, the plunger adapted for being inserted into the container, wherein the drive unit is configured to actuate a plug inserted into the container against the plunger for forcing the plug into the container and expelling liquid into the liquid passage; and
a needle, the needle being connected to the plunger and having a lumen extending therethrough, wherein the needle is adapted for extending through the plug to fluidly connect the liquid passage to the container via the lumen.

13. A method of dispensing a liquid and a gas from a tip for use in a medical procedure, the liquid held within a container, comprising the steps of;
selectively manipulating a single control unit to dispense only gas from the tip according to a second mode of operation;
selectively manipulating the single control unit to simultaneously dispense gas and liquid from the tip according to a third mode of operation; and
limiting the selective manipulation of the single control unit such that the second mode must occur before the third mode.

14. The method of claim 13 further comprising:
positioning a valve in a first position during the first mode, the first position fluidly disconnecting a gas supply conduit from a pneumatic drive unit and a gas passage;
positioning the valve in a second position during the second mode, the second position fluidly connecting the gas supply conduit only to the gas passage;
positioning the valve in a third position during the third mode, the third position fluidly connecting the gas supply conduit to the gas passage and the pneumatic drive unit; and
moving the valve only between first and third positions by way of the second position.

15. The method of claim 14 further comprising venting excess gas from the pneumatic drive unit to an exterior environment when the valve is in the first and second positions.
